# EUROPEAN PATENT APPLICATION

(11) **EP 1 122 538 A2**
(43) Date of publication of application: **08.08.2001**
(21) Application number: 01102021.1
(22) Date of filing: 30.01.2001
(51) Int. Cl.: G01N 33/36

(54) **Method and apparatus for checking and testing the characteristics of intervention clothes, in particular for firemen**

(30) Priority: 04.02.2000 IT VI000027
(71) Applicant: Lavanderie Dell'Alto Adige S.p.A., 39040 Ora (BZ) (IT)
(72) Inventor: Mumelter, Heinrich, 39100 Bolzano (IT); Bolognini, Riccardo, 58024 Massa Marittima (IT)
(74) Representative: La Ciura, Salvatore

(57) **Abstract**

The invention relates to a method and apparatus for checking the characteristics of "intervention" clothes (1) to ascertain whether the fabrics have undergone such changes and/or damages that they no longer have the safety characteristics which are provided for or set by law and thence have to be discarded.

The method of the invention comprises a first step of optically testing the external layer of the fabric, a second step of testing the reflectivity of the reflecting bands applied to the fabric, and finally a third stage of testing the capacity of heat insulation by means of infrared rays.

## Description

The present invention provides a method and apparatus for checking the characteristics of "intervention" clothes to ascertain that said clothes correspond to the laws in force and test if the safety requirements of the fabrics of "intervention" clothes, i.e. the clothes used for example by firemen, civil protection operators and those people who have to operate in dangerous conditions, in particular with fires or heat sources, have undergone possible decay after a certain period of use or after particular events or after washing operations or the like.

The method of the invention permits to ascertain whether the fabrics have undergone such changes and/or damages that they no longer have the safety characteristics which are provided for or set by law and thence have to be discarded.

The method of the invention comprises a first step of optically testing the external layer of the fabric, a second step of testing the reflectivity of the reflecting bands applied to the fabric, and finally a third stage of testing the capacity of heat insulation by means of infrared rays.

More in detail, the method of the invention comprises the steps of:
- framing simultaneously with a camera the garment and a board onto which a plurality of fabric portions are applied, said portions having been previously exposed to fire for a period and under conditions set by law to carry out destructive tests to determine the fabric characteristics;
- splitting up the garment image into a plurality of small areas;
- analysing the colour of each of these areas and comparing it with the colours on the board framed with the garment to test the fabric conditions;
- carrying out a similar operation with the bands of reflecting material;
- positioning into the fabric an inflatable dummy filled with a heated fluid;
- shooting the garment with an infrared camera while splitting up the garment image into a plurality of small areas;
- testing by infrared shooting the possible heat transfer for each area.

With the method and the apparatus of the invention it is possible to perform automatically all the operations of control of the "intervention" clothes, providing precise and repeatable results which cannot be obtained with conventional techniques and which, above all, do not need destructive tests.

As known, "intervention" clothes, e.g. the uniforms worn by firemen or by those people who have to operate in dangerous conditions, in particular with fire, are made of particular fabrics, generally comprising several layers of aramidic fabric, each of said layers having specific protection characteristics against fire, heat, etc.

These fabrics have to withstand high temperatures without burning or getting bored, they have to provide a heat insulation rate consistent with the conditions of use and they have also to provide the required strength.

Upon production these fabrics are subjected to very strict tests but, subsequently, the problem arises of checking, after a certain period of use or further to the use in particularly adverse environments, or more simply after washing operations, whether the clothes have kept the necessary characteristics.

Even though some characteristics can be analysed simply by a colorimetric analysis of the fabric, in other cases, for example when it is necessary to check the capacity of heat insulation of the fabric, destructive tests are performed which, of course, cannot be carried out regularly on all clothes.

Therefore, the need is felt in the field for means which permit to perform all necessary checks set by law without having to carry out destructive tests.

To this purpose the present invention provides a method and an apparatus for checking the fabrics of protective clothes, said method comprising the steps of shooting an image of the fabric by means of a camera, comparing the colour of the different fabric portions with the colours appearing on a board framed with the garment and bearing a plurality of fabric bands which have been previously exposed to fire for a period of time and under conditions set by law, carrying out a similar check for the reflecting bands and, finally, putting the fabric in touch with the heating element, e.g. a dummy filled with a heated fluid, and thence shooting an image of the garment by means of an infrared camera to ascertain the possible presence of areas which transfer more heat than set by law.

The present invention will now be described in detail, by way of non limiting example, with reference to the accompanying drawings in which:
- figure 1 schematically shows an apparatus for checking clothes according to the invention;

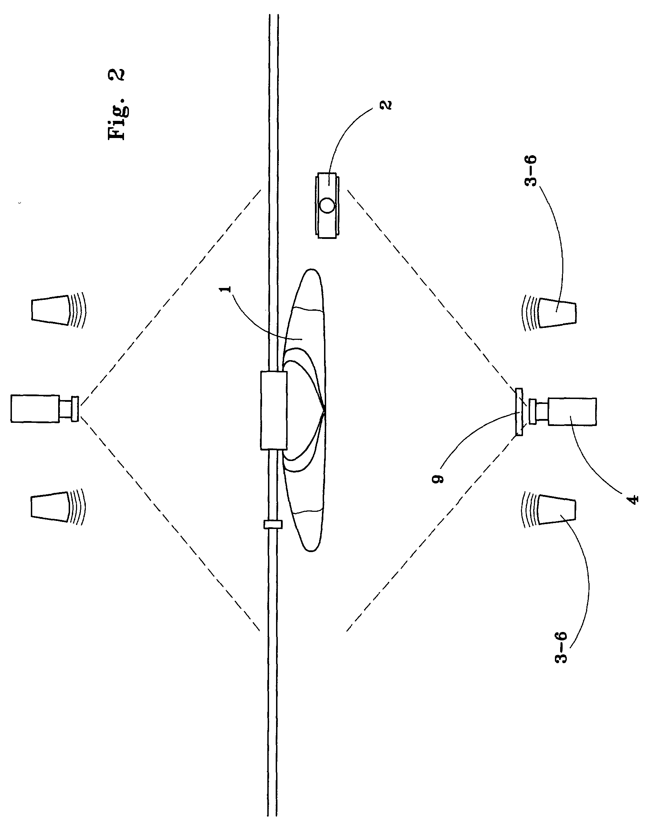

- figure 2 is a plan view of the apparatus of figure 1;
- figure 3 schematically shows a step of the clothes check with the method of the invention.

The method of the invention comprises a first step of checking the fabric conditions, by means of a colorimetric method consisting in shooting simultaneously with a camera the garment and a board showing the colours assumed by the fabric at the various temperatures.

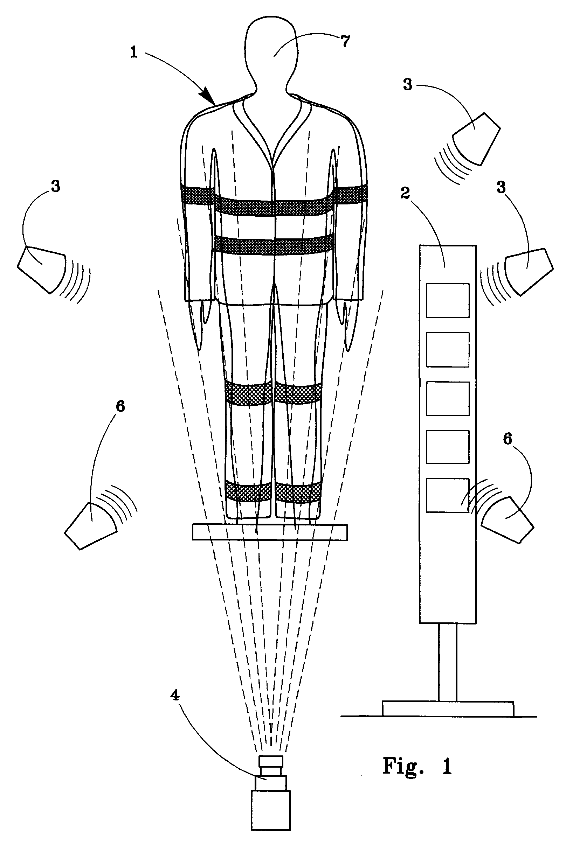

With reference to figure 1, numeral 1 shows the whole garment to be checked which is placed beside a board 2 with light sources 3 illuminating uniformly and simultaneously both the garment and the board, which are shooted by a camera 4.

From the camera sensor, e.g. a CCD sensor, the image is sent to a computer which, by suitable known programs, splits up the garment image into a plurality of small areas and analyses the fabric colour for each area. Subsequently, the program compares for each area the recorded colorimetric data with the data recorded on the board 2 by the same apparatus.

It is thus possible to test whether one or more areas of the garment have been subjected to high temperatures which might have compromised the fabric quality characteristics.

The result of this analysis can then be displayed in several ways, e.g. by showing the garment shape on a monitor and showing with a particular colour the presence of areas which have undergone changes.

Subsequently, the reflectivity of the reflecting bands 5 applied to this kind of clothes is tested.

To this purpose the bands are lit by means of conveniently inclined lamps 6, in particular positioned at about a 4° inclination. Also in this case the image shooted by the camera is analysed by the computer to check whether adjacent to the reflecting bands there are areas with poorer reflectivity characteristics.

The invention is also characterised in that it comprises a step in which the heat insulation properties of the fabric are checked by detecting the heat transfer by means of infrared rays.

To this purpose the garment is placed on a suitable support, in particular an inflatable dummy 7 which is then heated, for example by inflating it with a conveniently heated fluid.

The garment, possibly lit with infrared lights 8, is framed by a camera having a sensor which is sensitive to infrared rays, possibly placing an infrared screen 9 in front of the lens, in case the possible available light should be eliminated.

By this system the camera records a garment image in which the areas with greater heat transfer have a different density or colour with respect to the other areas.

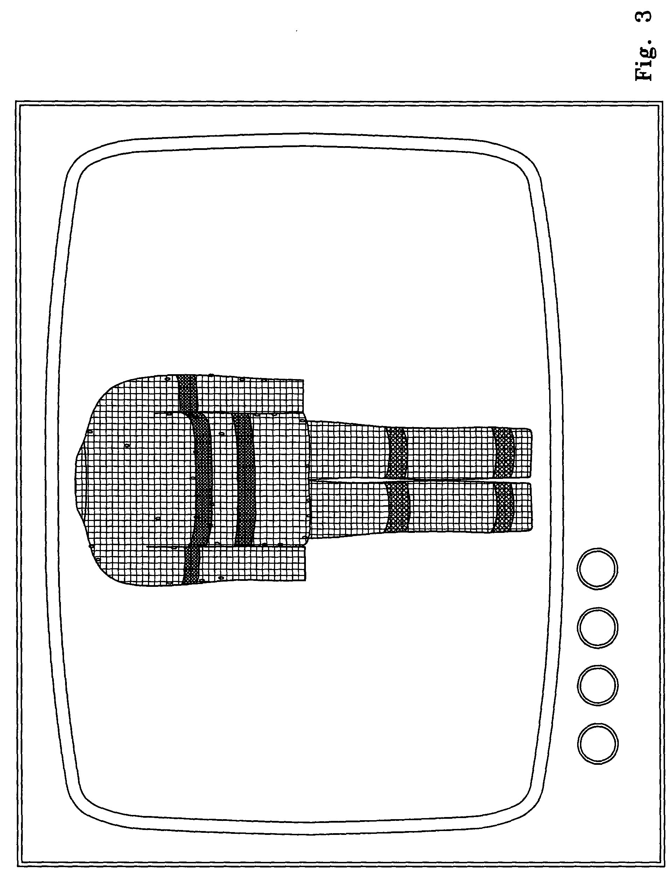

By this system, if the fabric or the internal stuffing have undergone changes or even localized decay, these will be immediately visible through the image shown on the monitor as different density and/or colour, as can be seen in figure 3.

The use of a heated dummy in association with the infrared reading of the garment image permits to check the garment conditions with a non destructive test which does not imply either any danger or drawback for the operators or any damage for the environment.

Therefore, the method of the invention permits to perform all necessary tests on "intervention" clothes quickly, cheaply and safely.

Those skilled in the art can then provide several modifications and variations, all of which, however, fall within the scope of the present invention.

## Claims

1. A method for checking the integrity of the fabrics of "intervention" clothes, characterised in that said method comprises at least a step in which:
• the garment is placed on a heated support;
• the garment is framed by an infrared camera;
• the heat insulation properties of the fabric are checked by detecting by means of said camera the heat flowing through the various fabric areas.

2. A method according to claim 1, characterised in that the signal detected by the camera sensor is sent to a computer in which:
• the garment shape is shown;
• the garment shape is split up into a plurality of areas having a predetermined extension;
• the heat flowing through each area is analysed;
• the amount of the heat flowing through each area is compared with a predetermined value;
• the areas in which the heat flow is greater than the allowable value are shown.

3. A method according to claim 1 or 2, characterised in that said garment is placed on an inflatable dummy which is heated by introducing fluid heated as necessary.

4. A method for checking the fabrics of "intervention" clothes according to claim 1, characterised in that said method comprises the following steps:
• reading the colour of the external layer of the fabric and comparing the colour with a board bearing samples of the same fabric which have been exposed to different temperatures;
• testing the reflectivity of the reflecting portions;
• placing a dummy into the garment and heating said dummy;
• showing, by means of infrared rays, the heat flowing through the different areas of the fabric.

5. A method according to claim 4, characterised in that in order to read the fabric and the samples positioned on the comparison board the same camera and the same light source are used.

6. An apparatus for checking the fabrics of "intervention" clothes, characterised in that it comprises:
• means for analysing the colour of the various fabric areas;
• means for comparing the detected colour with a colour board on which samples of the same fabric are applied, said fabric having been previously exposed to different temperatures;
• means for reading the heat flowing through the fabric in the different areas;
• means for showing the fabric areas in which the heat flow exceeds a predetermined level.

7. An apparatus according to claim 6, characterised in that it comprises means for testing the reflectivity of the reflecting areas of the fabric.

8. An apparatus according to claim 6 or 7, characterised in that said means for reading the fabric colour comprise a camera connected to devices processing the signal generated by the camera sensor, and in that said means for checking the heat flow through the fabric comprise a camera having an infrared screen.
